# EUROPEAN PATENT SPECIFICATION

(11) **EP 2 763 644 B1**
(45) Date of publication and mention of the grant of the patent: **21.06.2017**
(21) Application number: 12794479.1
(22) Date of filing: 02.10.2012
(51) Int. Cl.: A61H 5/00, G02C 7/14, A61B 3/028, G02C 7/02

(54) **DEVICE AND METHOD FOR FACILITATING THE IDENTIFICATION FROM A DISTANCE OF PREFERRED RETINAL LOCI (PRLS) AND THE ECCENTRIC FIXATION**
VORRICHTUNG UND VERFAHREN ZUR ERLEICHTERUNG DER IDENTIFIZIERUNG AUS EINER DISTANZ VON BEVORZUGTEN RETINALEN LOCI (PRLS) UND EXZENTRISCHE FIXIERUNG
DISPOSITIF ET PROCÉDÉ PERMETTANT DE FACILITER L'IDENTIFICATION À DISTANCE DE LOCUS RÉTINIENS PRÉFÉRÉS (LRP) ET LA FIXATION EXCENTRIQUE

(30) Priority: 07.10.2011 IT GE20110110
(43) Date of publication of application: 13.08.2014
(73) Proprietor: Iurilli, Michele, 16122 Genova (IT)
(72) Inventor: Iurilli, Michele, 16122 Genova (IT)
(74) Representative: Ausserer, Anton
(86) International application number: PCT/IB2012/055271
(87) International publication number: WO 2013/050926

(56) References cited:
- WO-A1-2007/138330
- US-A- 3 522 983
- US-A- 6 155 682
- R AL-KARMI ET AL.,: "Image relocation with prisms in patients with age-related macular degeneration", CAN J OPHTHALMOL, 2006, pages 313-318, XP002669261,

## Description

The present invention relates to a lens for people having a visual deficit in one or both the eyes comprising at least one magnifying portion and at least one prismatic portion with a predetermined prismatic element and a predetermined orientation, which orientation corresponds to an angle of rotation or of positioning of the prismatic element about an axis of rotation. The invention is defined by claims 1 and 6. Preferred embodiments are defined by the dependent claims. Further embodiments disclosed herein are for exemplary purpose only. In the usual everyday life images seen by people are composed of the light detected by the retina of the eyes of people, particularly of the fovea, the central region of the retina having the highest density of cones.

Some people have eye diseases that reduce the acuity of vision, the width and quality of the field of vision, related to problems involving the fovea, such as for example maculopathies, that is the formation of scotomas (blind spots), in the foveal region that do not allow light beams to be received, thus preventing information from being transmitted to the optic nerve and so reducing the acuity of vision of the individuals suffering from such diseases.

The displacement of the point of fixation in an area of the retina results therefrom, preferably in a parafoveal region, which does not have these blind spots, such to transmit as many information as possible through the optic nerve.

The presence of a scotoma in the foveal region leads to the loss of the foveal retinal fixation and to the development of an eccentric preferred retinal locus (PRL), an area inside which one tries to deflect the light beam, such to guarantee an area of fixation necessary for everyday visual tasks.

Such displacement is obtained by a deflection of the light beam impinging on the eye, which is mainly performed by using prismatic lenses.

Several types of glasses with one or both the prismatic lenses are known. Lenses provided in ordinary glasses, regardless of the type of defect to be corrected, such as myopia or astigmatism, can be combined with a portion of prismatic lens allowing the light beam impinging on the lens to be deflected, that is the light is deflected by means of the passage thereof through the prismatic element, creating a new point of fixation in the parafoveal region (PRL).

Such eccentric preferred retinal loci however are never at fixed and predetermined positions, they can be different and the fact of identifying them is not immediate and it requires a training by the patient.

The known prior art magnifying prismatic lenses have a fixed orientation namely they provide the prismatic element always at a specific position that therefore deflects the light beam always in the same direction.

Therefore there is the unsatisfactory need of creating glasses in combination with prismatic lenses that can be adapted to different patients, having different clinical situations and that can be further associated to an efficient method for defining the correct type of prismatic lens, as well as for correctly defining the eccentric preferred retinal loci.

The present invention achieves the above aims by providing a lens for people having a visual deficit as described above, wherein the prismatic element is rotated and/or rotatable by a predetermined angle about an axis of rotation, which axis of rotation is parallel and/or coincident with the axis perpendicular to the anatomical frontal plane of said lens, said angle being defined on the basis of the position of the eccentric preferred retinal locus (PRL) within the parafoveal region in order to allow the image to be fixed on said area.

The rotation of the prismatic element allows the light beam to be deflected in any area of the retina and the angle of rotation will be such that the light beam will be deflected inside the specific eccentric preferred retinal locus (PRL) depending on the subjective characteristics of the visual disease.

Thus a lens is made which has a magnifying portion in combination with a portion of prismatic lens.

It has to be noted that due to the specific configuration described, the prismatic element is rotatable not only about the axis of rotation parallel and/or coincident with the axis perpendicular to the front anatomical plane of the lens, but also with respect to the magnifying portion.

Advantageously both the portions the prismatic portion and the magnifying one are rotatable one with respect to the other about an axis of rotation parallel and/or coincident with the axis perpendicular to the front anatomical plane of said lens.

The lens obtained in this manner, besides having the prismatic effect will have all the known characteristics of the lenses of the usual glasses, such as for example the possibility of obtaining a correction of the refractive error and of being antireflection and scratch-resistant.

The present invention relates also to glasses for people having a visual deficit in one or both the eyes, which glasses comprise a pair of lenses of which pair at least one is made with the characteristics of the lens described above.

Similarly the glasses object of the present invention can provide lenses made of glass material and of polycarbonate or of any material known in the prior art.

Preferably the lens described above can provide an arrangement comprising a photoselective filtering colour so called "blue blocker", intended to protect the ocular means from UV rays and from blu light, such to increase the contrast and therefore to optimize the visual ability.

According to an improvement, in combination with the glasses object of the present invention there can be provided means for regulating said angle.

Such aspect is particularly advantageous since it is possible to regulate and change the focusing point, covering all the eccentric preferred retinal loci (PRL) of the patient.

Such regulating means can be composed of manual regulating means known in the prior art and provided in glasses for performing visual tests used by opticians and ophthalmologists.

As an alternative such regulating means can be of the automated type, for example electronically-controlled ones, for regulating the angle of rotation of the prismatic element or through inputs sent from a remote controller or from the patient or from the physician.

It has to be pointed out that the present invention relates to glasses for performing visual tests in combination with lenses described above, of the type used by opticians and ophthalomologists.

Similarly the present invention relates to glasses used in everyday life, in combination with lenses described above.

Since visual defects, such as a central or paracentral scotoma for example due to a maculopathy, can involve both the eyes of the same patient, advantageously both the lenses of the glasses object of the present invention are made of such materials and with such curves to generate a magnification in combination with a prismatic lens portion.

In this case it is possible to provide the orientation of the prismatic element to be different depending on the lens: such aspect is particulary advantageous since the formation of spots can occur in different retinal areas, depending on the individual and on the pathology, therefore the healthy retinal areas, that remain in eccentric areas with respect to the fovea and which are self-defined by the individual suffering from retinal disease as "preferred" ones, that is alternative to the damaged foveal area, can be in different positions and retinal areas that consequently require different deflections of the image projection, therefore by means of different angles of the prismatic element.

According to one embodiment, the lenses object of the present invention can have different graduations, that is they can provide any measure both as regards spherical cylindrical dioptres and prism dioptres.

With a particular reference to prism dioptres each lens can have a graduation ranging from 1 to 14 prism dioptres, preferably from 2 to 10 prism dioptres, particularly ranging from 3 to 8 prism dioptres.

Three different types of lenses are typically made having 3, 4, 5, 6 and 8 prism dioptres respectively.

Defining the proper orientation of the prismatic element and the correct type of lens to be used is an essential aspect which is strictly related to the eccentric preferred retinal loci (PRL) and a method for the qualitative and/or quantitative evaluation of the paracentral visual field, for the identification of the PRLs from a distance and of the projection of the scotomatous areas, as well as the tools necessary for performing such methods.

Thus advantageously both the lens and the glasses object of the present invention can be provided in combination with such methods, as well as the tools necessary for performing them, which all are the object of the present invention.

Therefore advantageously the invention relates also to an optotype for the qualitative and quantitative evaluation of the paracentral visual field, for the identification of PRLs from a distance and of the projection of the scotomatous areas composed of a circular element providing a graphical representation composed of a plurality of concentric circles and an alphanumeric character and/or a symbol placed in the center of the concentric circles.

Moreover there is provided a plurality of lines arranged along the radii of the concentric circles at regular intervals, such that each concentric circle is divided into a plurality of sectors.

The size of the alphanumeric character and/or the symbol, as well as the size of each radius of the concentric circles are defined on the basis of the distance between the optotype and the patient the visual test of which has to be performed.

With a particular reference to patients suffering from visual diseases of the maculopathy type, such type of optotype allows the position, the size and the quality of the non-visual area to be verified. Moreover below it will be described how the optotype object of the present invention allows the power and the orientation of the prismatic elements of the lenses belonging to the glasses of the present invention to be defined.

Preferably the size of the alphanumeric character and/or symbol is defined such to define a visual acuity of 1/10 based on the distance of the patient.

According to a preferred embodiment the optotype of the present invention provides three concentric circles.

The size of the radii of the circles occurs in such a way that the size of the innermost circle is equal to the displacement of a light beam generated by a number of prism dioptres ranging from 3 to 5, in particular 4 prism dioptres, while the size of the radius of the second circle is equal to the displacement of a light beam generated by a number of prism dioptres ranging from 5 to 7, particularly 6 prism dioptres, and the size of the radius of the third circle is equal to the displacement of a light beam generated by a number of prism dioptres ranging from 7 to 9, particularly 8 prism dioptres.

These particular sizes are such to define the proper type of lenses, that is to define which is the best lens among those described above, the 4, 6 or 8 prism dioptre one.

According to an improvement the optotype object of the present invention has an alphanumeric character and/or symbol at the outermost concentric circle and at each line.

Such improvement will allow, as it will be widely described below, the proper angle of the prismatic element to be defined on the basis of the visual defects of the patient.

Moreover advantageously the plurality of sectors defined by the intersection between the lines and the concentric circles can have at least partially different colours, or filling elements that allow a sector to be differentiated with respect to another one.

The division into sectors with different colours serves for evaluating how the vision of the patient changes as the prism dioptres increase and decrease, for dividing the whole area of the optotype in order to facilitate the work of the operator performing the visual acuity examination and it allows the identification of the non-visual area to be facilitated for people having cognitive problems.

It has to be noted that the optotype of the present invention can be provided in combination with an electronic system for performing visual tests.

It is possible to obtain the graphical representation of the optotype of the present invention through a computer-based system, having all the characteristics of the current systems used for performing the examination of the visual field.

As said above, the present invention relates also to a method for the qualitative and quantitative evaluation of the paracentral visual field, for the identification of the PRLs from a distance and of the projection of the scotomatous areas by using glasses for people having a visual deficit in one or both the eyes as described above.

Such glasses comprise a pair of lenses, of which pair of lenses, at least one lens is shaped such to provide the necessary optic correction, comprising a prismatic portion with a predetermined prismatic element and a predetermined orientation, which orientation corresponds to an angle of rotation or of positioning the prismatic element, about an axis of rotation.

The method of the present invention provides the identification of PRLs from a distance to be performed through the rotation of the prismatic element about an axis of rotation, which is parallel and/or coincident with the axis perpendicular to the anatomical frontal plane of the lens.

After rotating the prismatic element it is preferable to perform a check step for verifying whether this rotation has actually led to a facilitation in identifying the PRL from a distance and therefore to an increase in the visual acuity of the patient.

Advantageously such check step is made by using the optotype described above.

According to a particular embodiment the method just described provides one or more steps replacing the prismatic lens with prismatic lenses having a different dioptre, it being provided after each replacement step a further check step performed with the lens having a different dioptre used.

Due to the synergy and above all the correlation between the lens, the glasses, the optotype and the method for the qualitative and quantitative evaluation of the paracentral visual field, for the identification of the PRLs from a distance and of the projection of the scotomatous areas the present invention further relates to a kit for performing such evaluation.

The kit of the present invention provides a lens for people having a visual deficit in one or both the eyes, in combination with means checking the qualitative and quantitative evaluation of the visual field intended to correct the size of the angle of rotation of the prismatic element.

The lens provided in said kit is a lens as described above and it has all the characteristics related to the already discussed lenses.

Similarly the check means can be composed of an optotype made according to one or more of the characteristics individually or in combination related to the optotype of the present invention.

The kit object of the present invention can be provided in combination with the type of glasses described above.

From what discussed above, it is clear how the use of a lens and/or glasses for people having a visual deficit made according to one or more of the characteristics claimed above in combination with a method performed by the described steps can lead to the achievement of a training method for the search and fixation of the eccentric preferred retinal locus (PRL) in the parafoveal area in people suffering from visual diseases, which also is an object of the present invention.

Similarly such training method can be provided in combination with the optotype described above.

Advantageously such training method can be used as a training method for rehabiliting peripheral preferred retinal areas (PRLs) from a distance.

The training method for the search and fixation of the eccentric preferred retinal locus (PRL) object of the present invention, is also a potential tool useful for research in neurologic field, since the use of the glasses object of the present invention would allow doses of photonic stimuli to be intensively and not invasively administered, which would allow sufficient data to be generated for measuring, evaluating the sensitivity and for verifying the plasticity of the central nervous system.

According to an improvement the training method of the present invention can therefore be used as a method for stimulating peripheral retinal preferred loci (PRLs) as a research/experimental tool intended to study the neuronal response.

Finally the training method object of the present invention can be used as a method for performing stimulations in the retinal areas in such an amount and intensity sufficient to allow objective data to be generated which are sufficient for evaluating and verifying the brain sensitivity and plasticity, useful also for example for functional neuroimaging studies.

These and other characteristics and advantages of the present invention will be more clear from the following description of some embodiments shown in annexed drawings wherein:
figs. 1a and 1b are a lens object of the present invention according to a possible embodiment, according to a side view and a perspective view respectively;
fig. 2a is the optotype object of the present invention according to a possible variant embodiment;
fig. 2b is the optotype object of the present invention according to a further variant embodiment;
fig. 3 is a possible arrangement of the method for measuring the visual acuity object of the present invention.

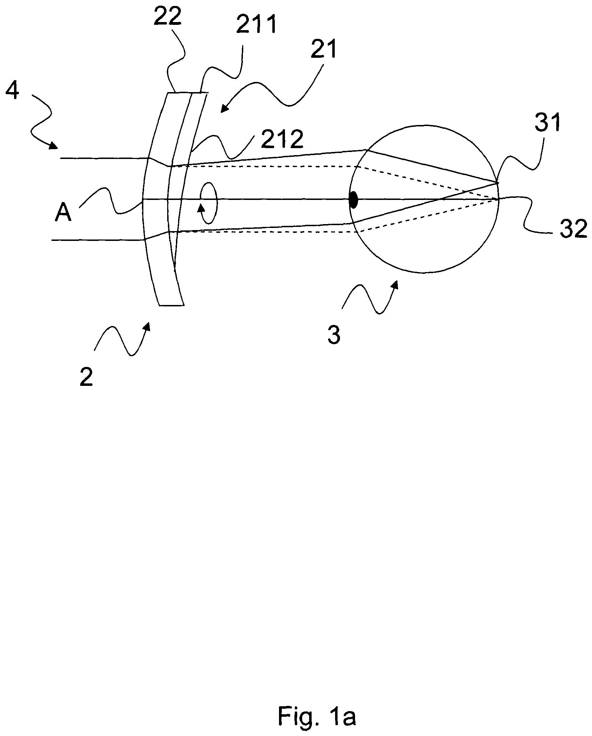

Figure 1a shows a lens 2 for people having a visual deficit in one or both the eyes object of the present invention, made according to a possible embodiment.

The lens 2 is shaped such to provide the necessary optical correction and it comprises a prismatic portion having a predetermined prismatic element 21 with a predetermined orientation corresponding to an angle of rotation or of positioning of the prismatic element 21 about an axis of rotation A.

The prismatic element 21 is rotated by a predetermined angle about the axis of rotation A, which is parallel and/or coincident with the axis perpendicular to the anatomical frontal plane of the lens 2.

The angle of rotation is defined on the basis of the position of the eccentric preferred retinal locus (PRL) 31 within the parafoveal region in order to facilitate image fixation on said area 31.

Figure 1a shows a lens 2 with the prismatic element 21 able to rotate about an axis of rotation A.

The prismatic element 21 is composed of a base 211 and a side surface 212 and it is rotated by a predetermined angle about the axis of rotation A, which is parallel and/or coincident with the axis perpendicular to the anatomical frontal plane of the lens 2.

The prismatic element 21 allows light beams 4 impinging on the outer surface of the lens 2 to be deflected and this deflection causes the light beam to be directed in an area near the fovea 32 of the eye 3.

The size of the angle of rotation of the prismatic element 21 is such that the area where the light beam is deflected coincides with the eccentric preferred retinal locus (PRL) of the eye 3.

Particularly in figure 1a dashed lines show the path of the light beam 4 without the effect of the prismatic element 21, while continuous lines show the path of the light beam 4 deflected by the prismatic element 21.

It is clear that by changing the orientation of the prismatic element 21 it is possible to change the projection of the image on a different position of the eccentric preferred retinal locus (PRL) 31 within the parafoveal region, that is it is possible to identify different areas within the parafoveal region intended to be used as eccentric preferred retinal loci.

Moreover the rotation of the prismatic element 21 along a whole circumference, that is with angles with a size ranging from 0 to 360°, allows the point 31 to be displaced along a corresponding circumference in the parafoveal region.

Should it be necessary to displace the point 31 not only along a circumference it is sufficient to change the power of the prismatic element 21 for displacing the point 31 in any area of the retina belonging to the eye 3.

According to a preferred embodiment the lens 2 comprises a magnifying lens 22 in combination with a portion of prismatic lens 21.

According to an embodiment it is possible to provide means for regulating the angle of rotation about the axis A of the prismatic element 21.

In this case the pair of lenses would be composed of a first lens and a second lens, composed of a magnifying lens 22 in combination with a portion of prismatic lens 21.

Advantageously the size of the angle of rotation of the prismatic element 21 can be different in the two lenses and it will be always defined on the basis of the positioning of the eccentric preferred retinal loci of the patient.

It will be clear below, particularly in the description of the following shown examples, how such angle is defined and with which means.

The lenses 2 object of the present invention can be of any graduation, but advantageously the prismatic lens provides a graduation ranging from 1 to 14 prism dioptres, preferably from 2 to 10 prism dioptres, in particular ranging from 3 to 8 prism dioptres.

According to a preferred variant embodiment there are provided at least 5 types of lenses 2, which prismatic lenses provide a graduation equal to 3, 4, 5, 6 and 8 prism dioptres respectively.

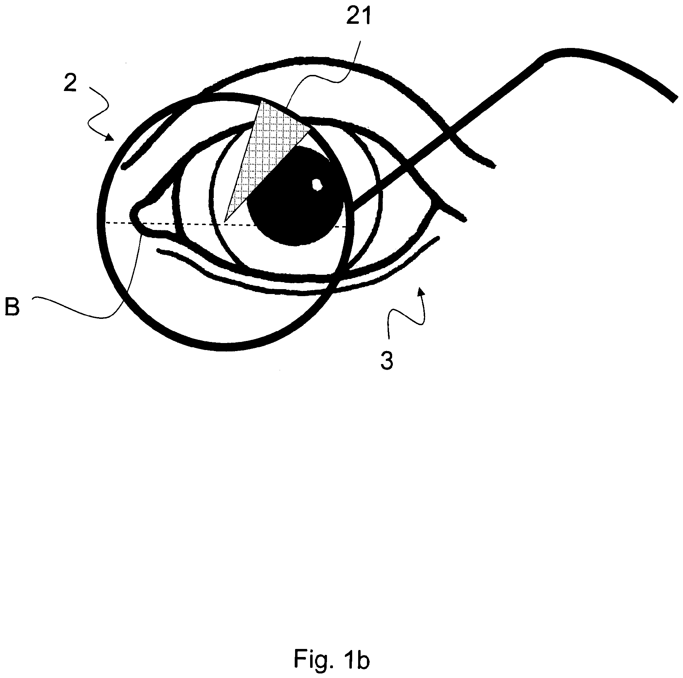

Figure 1b shows a perspective view of the lens 2, where it is shown that the prismatic element 21 is angled by a predetermined angle with respect to the horizontal axis B of the lens.

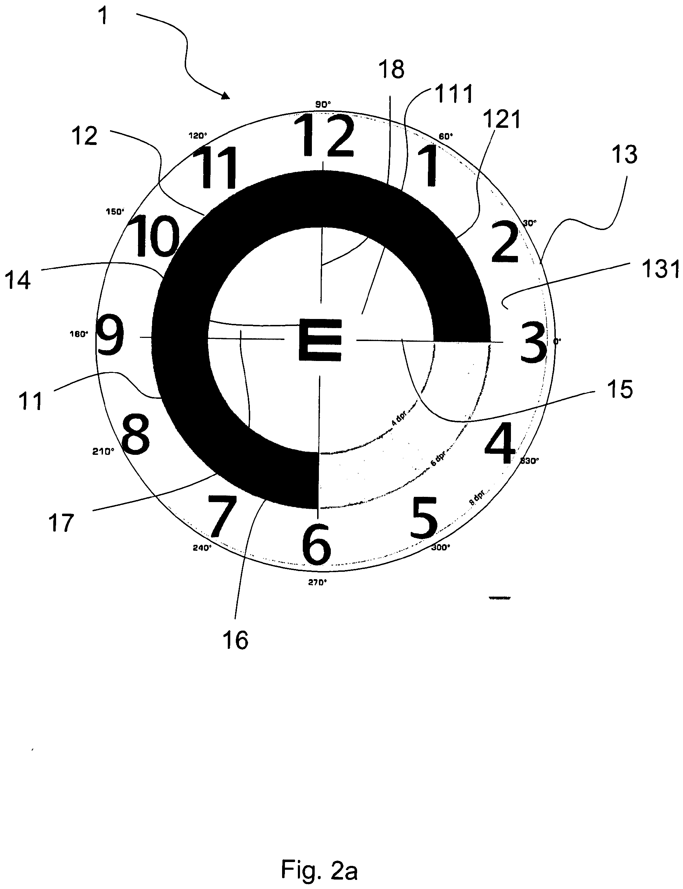

Figure 2a shows the optotype for the qualitative and quantitative evaluation of the paracentral visual field, for the identification from a distance of PRLs and of the projection of the scotomatous areas object of the present invention according to a first embodiment.

The optotype is composed of a circular element 1 providing a graphical representation composed of a plurality of concentric circles 11, 12 and 13 and of an alphanumeric character 14 placed at the centre of the concentric circles 11, 12 and 13.

There is further provided a plurality of lines 15, 16, 17 and 18 arranged along the radii of the concentric circles 11, 12 and 13 at regular intervals, such that each concentric circle is divided into a plurality of sectors 111, 121, 131.

The size of the alphanumeric character 14, as well as the size of each radius of the concentric circles 11, 12 and 13 are defined on the basis of the distance between the patient whose visual acuity has to be measured and the optotype.

Particularly the size of the alphanumeric character 14 is determined such to define a visual acuity of 1/10 on the basis of the patient's distance.

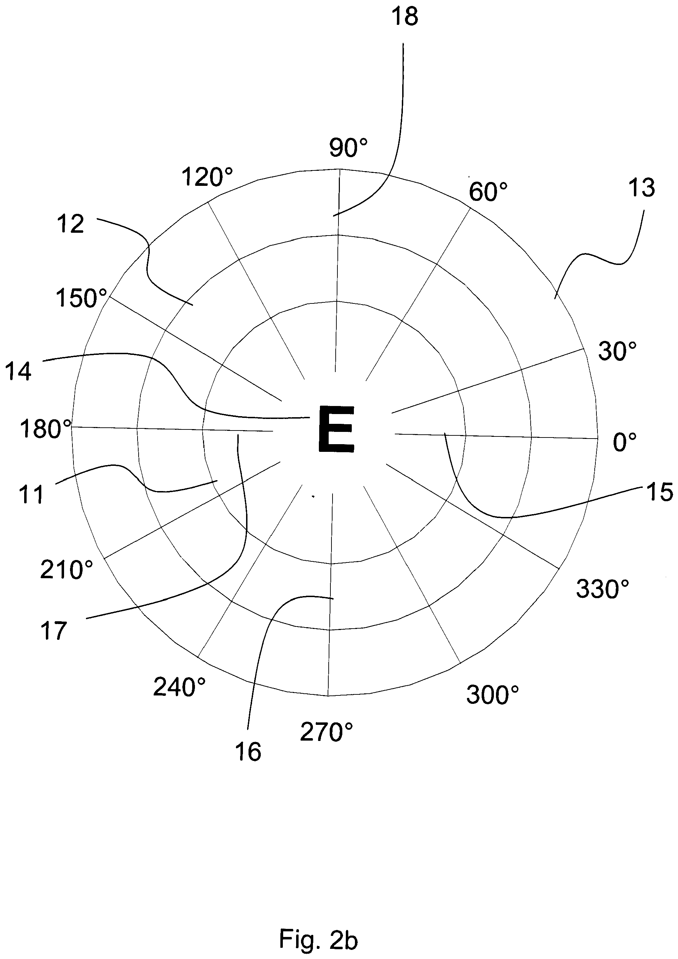

The character shown in figures 2a and 2b is an "E" font "sloan" used for visual tests.

With a particular reference to figures 2a and 2b, the shown optotype has measures that are in scale, for illustrative simplicity, with respect to an optotype whose measures are calculated for the positioning of the patient at 3-metre distance.

As it will be explained below it will be easy to obtain the shown optotype for any distance of the patient therefrom, making similar optotypes but with different measures which are all the object of the present invention.

Advantageously three concentric circles 11, 12 and 13 are provided.

The size of the radius of the circle 11 is a number equal to the displacement of a light beam generated by a number of prism dioptres ranging from 3 to 5, particularly 4 prism dioptres.

The size of the radius of the circle 12 is a number equal to the displacement of a light beam generated by a number of prism dioptres ranging from 5 to 7, particularly 6 prism dioptres.

The size of the radius of the circle 13 is a number equal to the displacement of a light beam generated by a number of prism dioptres ranging from 7 to 9, particularly 8 prism dioptres.

With a particular reference to figure 2a the sizes of the radii of circles 11, 12 and 13 are calculated on the basis of a patient placed three metres from the optotype.

Since it is known that a prism dioptre displaces an image of one centimetre at a distance of one metre, in the case of the radius of the first circle 11 the prism dioptres of interest are 4 at the distance of 3 metres, therefore the radius of the first circle 11 will be equal to 12 cm.

Similarly the radius of the circle 12 will be equal to 18 cm, while the radius of the circle 13 will be equal to 24 cm.

Since the step from one circle to the other one provides a jump of 2 prism dioptres, the areas comprised between the circle 11 and the circle 12 and between the circle 12 and the circle 13 respectively will have a thickness equal to 6 cm (that is 2 prism dioptres for a 3-metre distance).

Moreover there are provided 12 numbers placed as the numbers on the dial of the watch and arranged between the circumference of the circle 13 and the circumference of the circle 12, having the same size of the alphanumeric character 14.

Moreover at the outside of the circle 13 there are provided other numbers and symbols which are used by the operator performing the visual test and that mainly denote the angle according to which the prismatic element of the lens belonging to the glasses of the present invention has to be oriented.

With a particular reference to figure 2a, the optotype of the present invention has four lines 15, 16, 17 and 18 placed with reference to the watch dial, at 12 o'clock, at 3 o'clock at 6 o' clock and at 9 o'clock respectively.

Such lines divide the area comprised between the circumference of the first circle 11 and the circumference of the second circle 12 into 4 different sectors which are filled with different colours or with different filling elements, such as shown in figure 1.

Regardless of what is filled, the differentiation of the 4 sectors is desired, since it has several advantages among which a delimitation of the displacement of the image with 4 prism dioptres and with 6 prism dioptres, an identification of 4 macro-areas of the optotype, in order to facilitate the work of the operator and to facilitate the identification of the non-visual area for people having cognitive problems.

On the contrary figure 2b shows the optotype of the present invention according to a further embodiment, wherein there is provided a higher number of different lines with respect to the previous version which are also positioned at regular intervals along the radii of the three concentric circles 11, 12 and 13.

Such lines allow the size and the topography of the non-visual area of the patient to be defined more accurately. Unlike the previous version only the alphanumerical character 14 is maintained, the numbers placed like on a watch dial have been eliminated and the only references that indicate how orienting the prismatic element are given by lines and numbers, showing the angle degrees, provided outside the circle 13.

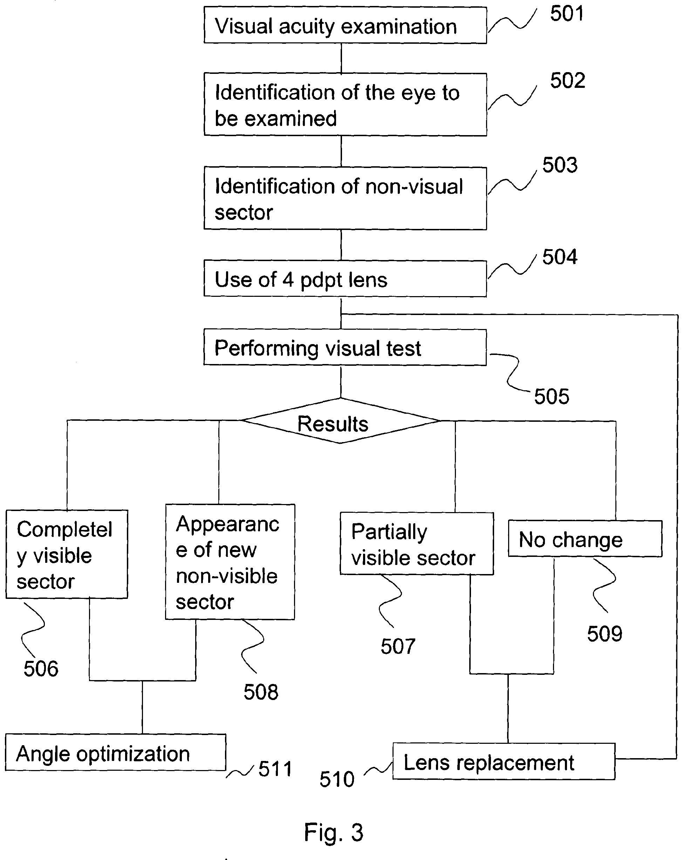

Figure 3 shows a possible arrangement of the method for the qualitative and quantitative evaluation of the paracentral visual field, the identification from a distance of PRLs and the projection of the scotomatous areas of the present invention.

Due to the characteristics described up to now with reference to the glasses and the optotype object of the present invention, it is clear that the method described below not only allows a qualitative quantitative evaluation of the paracentral visual field, the identification from a distance of PRLs and the projection of the scotomatous areas, but it can also be a useful training method for the search and fixation of the eccentric preferred retinal locus (PRL) within the parafoveal region of patients, as well as, by the use of the optotype, it can be a method for the correct prescription of prismatic lenses.

Particularly the method for the qualitative quantitative evaluation of the paracentral visual field, the identification from a distance of PRLs and the projection of the scotomatous areas therefore provides the use of glasses for people having a visual deficit in one or both the eyes comprising a pair of lenses, of which pair of lenses, at least one lens 2 is shaped such to provide the proper necessary optical correction.

Such lens 2 is preferably made according to the characteristics shown in figure 1, that is it comprises a prismatic portion with a predetermined prismatic element 21 and a predetermined orientation, corresponding to an angle of rotation or of positioning of the prismatic element 21 about the axis of rotation (A).

The qualitative and quantitative evaluation of the paracentral visual field, the identification from a distance of PRLs and the projection of the scotomatous areas occur by rotating the prismatic element 21 about the axis of roation (A), which is parallel and/or coincident with the axis perpendicular to the anatomical frontal plane of the lens 2.

Preferably there is provided a check step intended to check that said prismatic element 21 has been rotated such to facilitate the eccentric fixation and therefore to increase the visual acuity of the patient.

Advantageously such check step is performed by control means which are composed of the optotype and of the prismatic lenses described above.

According to an improvement of the method object of the present invention there are provided one or more steps for replacing the prismatic lens with prismatic lenses having a different dioptre, there being provided after each replacement step a further check step performed with the lens with the different dioptre used.

Particularly figure 3 shows the method steps just described.

It has to be pointed out that such steps refer to the type of lenses with 4, 6 and 8 prism dioptres, but they can be adapted to any dioptre.

At first the visual acuity of the patient is analysed, denoted by 501, which can be made both with the correction currently used by the patient, and without the correction.

If one realizes that the correction currently used by the patient is not the optimal one, it is possible to optimize such correction by changing the graduation by the lenses known in the prior art.

Then in step 502 the eye is identified on which the qualitative and quantitative evaluation test of the paracentral visual field, the identification from a distance of PRLs and the projection of the scotomatous areas has to be performed.

The choice of the eye does not change the method steps that will be described below, it is possible to perform the test on both the eyes, by analysing one eye at a time.

Then at step 503 the optotype described above is used and at least one area, a sector of the optotype not visible by the patient is identified and the angle corresponding to the non-visual area is identified by the optotype.

For example with a particular reference to figure 2a, if the patient is not able to see the number "1", the angle identified will be 60°.

At step 504 a lens having 4 prism dioptres is used, such that the prismatic element 21 is rotated by 60°, that is its base 211 is oriented at the non-visual sector.

Then, at step 505, the visual test of the optotype is again performed and one analyses whether the previously non-visible sector has become visible.

Therefore 4 alternative solutions are possible:
the previously non-visible sector now is completely visible, 506;
the previously non-visible sector now is partially visible, 507;
the previously non-visible sector now is completely visible, but another non-visible sector is found, 508;
no change has occurred, 509.

In cases 507 and 509 a step 510 follows which provides the replacement of the 4 prism dioptre lens with the 6 prism dioptre lens which has the prismatic element oriented in the same direction as the previous one.

The visual test provided at step 505 is repeated with the 6 prism dioptre lens and if steps 507 and 509 are repeated a further replacement is performed by using a 8 prism dioptre lens.

In cases 506 and 508 a step 511 follows where the angle of the prismatic element 21 is improved.

Firstly the prism is rotated by 180° with respect to the angle at step 504, then rotations by 90° and 270° are made, still with respect to the angle at step 504.

The visual test is repeated and the response of the patient at each rotation is verified, the rotation allowing the best optimization of the visual field by the patient is identified, then starting from such condition it is possible to perform a fine correction, by rotating the prism by smaller angles, such as for example 10°, 20°, 30°. 330°, 340°, 350°.

Finally it is possible to perform the test even on the other eye, then to continue the visual test once the vision of both the eyes is corrected.

Once the lens object of the present invention, the optotype and the method are described, it is clear how by modifying the lens, the thickness and the angle of the prismatic element 21 it is possible to displace the point 31 on the whole surface of the retina of the eye 3.

The graphical representation 1 of the optotype can correspond to the retinal area inside which the point 31 is displaced.

Therefore depending on the blind region identified by the patient inside the optotype it will be possible to displace the point 31 on the retina such that the patient can focus the light beams in an area of the retina not suffering from maculopathy.

## Claims

1. Lens for people having a visual deficit in one or both the eyes comprising at least one magnifying portion and at least one prismatic portion with a predetermined prismatic element (21) and a predetermined orientation, which orientation corresponds to an angle of rotation or of positioning of said prismatic element about an axis of rotation (A),
**characterized in that**
said prismatic element (21) is rotated and/or rotatable by a predetermined angle about an axis of rotation (A), which axis of rotation (A) is parallel and/or coincident with the axis perpendicular to the anatomical frontal plane of said lens (2),
said angle being defined on the basis of the position of the eccentric preferred retinal locus (PRL) (31) within the parafoveal region in order to facilitate image fixation on said area (31).

2. Lens according to claim 1, wherein said at least one prismatic portion provides a graduation ranging from 1 to 14 prism dioptres, preferably from 2 to 10 prism dioptres, particularly ranging from 3 to 8 prism dioptres.

3. Glasses for people having a visual deficit in one or both the eyes comprising a pair of lenses, **characterized in that** at least one lens of said pair of lenses is made according to one or more claims 1 or 2.

4. Glasses according to claim 3, wherein said pair of lenses is composed of a first lens and a second lens, which first lens and which second lens are composed of a magnifying portion (22) in combination with a prismatic portion (21),
the angle of rotation of the prismatic element (21) of said first prismatic lens having a measure different than the angle of rotation of the prismatic element (21) of said second prismatic lens.

5. Glasses according to claims 3 or 4, wherein means for regulating said prismatic element (21) are provided.

6. Method for the qualitative and quantitative evaluation of the paracentral visual field, for the identification of the eccentric preferred retinal loci (PRLs) from a distance and of the projection of the scotomatous areas by using at least one lens according to claim 1,
further **characterized in that**
the qualitative and quantitative evaluation of the paracentral visual field, the identification from a distance of PRLs and of the projection of the scotomatous areas occurs by rotating said prismatic element (21) about an axis of rotation (A).

7. Method according to claim 6, wherein there is provided a check step intended for checking that said prismatic element (21) has been rotated such to facilitate the eccentric fixation and therefore to increase the visual acuity of the patient, said check step being performed by using an optotype that is composed of a circular element providing a graphical representation (1) composed of a plurality of concentric circles (11, 12, 13) and of an alphanumeric character (14) and/or a symbol placed at the centre of said concentric circles (11, 12, 13), there being provided a plurality of lines (15, 16, 17) arranged along the radii of said concentric circles (11, 12, 13) at regular intervals, such that each concentric circle is divided into a plurality of sectors (111, 121, 131), the size of said alphanumeric character (14) and/or of said symbol, as well as the size of each radius of said concentric circles (11, 12, 13) being defined on the basis of the distance between the patient and said optotype.

8. Method according to one or more of the preceding claims 6 and 7, wherein there are provided one or more steps for replacing said at least one prismatic lens with prismatic lenses having a different dioptre, there being provided after each replacement step a further check step performed with the lens with the different dioptre used.

9. Kit for the qualitative and quantitative evaluation of the paracentral visual field, for the identification from a distance of the PRLs and of the projection of the scotomotous areas **characterized in that** it provides a lens according to claim 1, and it further comprises checking means intended for correcting the measure of the angle of rotation of said prismatic element (21).

10. Training method for the search and fixation of the eccentric preferred retinal locus (PRL) within the parafoveal region **characterized in that** it provides to use a lens made according to one or more of the preceding claims 1 or 2.

## Patentansprüche

1. Linse für Menschen mit einer ein- oder beidseitigen Fehlsichtigkeit, welche mindestens einen vergrößernden Bereich und mindestens einen prismatischen Bereich mit einem vorbestimmten prismatischen Element (21) und einer vorbestimmten Ausrichtung vorweist; diese Ausrichtung entspricht einem Rotationswinkel oder einem Positionswinkel des prismatischen Elements um eine Rotationsachse (A),
**dadurch gekennzeichnet, dass**
das prismatische Element (21) mit einem vorbestimmten Winkel um eine Rotationsachse (A) gedreht bzw. drehbar ist; diese Rotationsachse (A) ist parallel zur und/oder fällt mit der Achse zusammen, die senkrecht zur anatomischen Frontalebene der Linse (2) verläuft;
der Winkel wird anhand der Lage des exzentrischen bevorzugten Netzhautareals (PRL) (31) innerhalb des parafovealen Bereichs definiert, um die Bildfixierung in diesem Bereich (31) zu erleichtern.

2. Linse nach Anspruch 1, bei der dieser mindestens eine prismatische Bereich eine Graduierung aufweist, die zwischen 1 und 14 Prismendioptrien liegt, vorzugsweise zwischen 2 und 10 Prismendioptrien, insbesondere zwischen 3 und 8 Prismendioptrien.

3. Brille für Personen mit einer ein- oder beidseitigen Fehlsichtigkeit, die ein Paar Linsen hat, das **dadurch gekennzeichnet ist, dass** mindestens eine der Linsen nach einem oder mehreren der Ansprüche 1 oder 2 gefertigt ist.

4. Brille nach Anspruch 3, bei der das Paar Linsen aus einer ersten Linse und einer zweiten Linse besteht; die erste Linse und die zweite Linse bestehen aus einem vergrößernden Bereich (22) in Kombination mit einem prismatischen Bereich (21);
der Rotationswinkel des prismatischen Elements (21) der ersten prismatischen Linse unterscheidet sich in der Größe von dem Rotationswinkel des prismatischen Elements (21) der zweiten prismatischen Linse.

5. Brille nach Anspruch 3 oder 4, bei der Mittel zur Anpassung des besagten prismatischen Elements (21) vorgesehen sind.

6. Methode für die qualitative und quantitative Untersuchung des parazentralen Gesichtsfelds, für die Bestimmung aus einer Entfernung von exzentrischen bevorzugten Netzhautarealen (PRLs) und der Projektion der von Skotomen betroffenen Bereiche durch die Nutzung von mindestens einer Linse nach Anspruch 1, ferner
**dadurch gekennzeichnet, dass**
die qualitative und quantitative Untersuchung des parazentralen Gesichtsfelds, die Bestimmung aus einer Entfernung der PRLs und der Projektion der von Skotomen betroffenen Bereiche durch das Rotieren des prismatischen Elements (21) um eine Rotationsachse (A) erfolgt.

7. Methode nach Anspruch 6,
bei der ein Prüfschritt vorgesehen ist, welcher der Überprüfung dessen gilt, dass das prismatische Element (21) so gedreht wurde, dass es die exzentrische Fixation erleichtert und somit die Sehschärfe des Patienten verbessert;
dieser Prüfschritt wird unter Einsatz einer Optotype durchgeführt, die aus einem kreisförmigen Element besteht, das eine grafische Darstellung (1) liefert, die aus einer Vielzahl von konzentrischen Kreisen (11, 12, 13) und aus einem alphanumerischem Zeichen (14) und/oder einem Symbol besteht, das in der Mitte der konzentrischen Kreise (11, 12, 13) platziert ist; vorhanden ist auch eine Vielzahl von Linien (15, 16, 17), die entlang der Radien dieser konzentrischen Kreise (11, 12, 13) in regelmäßigen Abständen so angeordnet sind, dass jeder konzentrische Kreis in eine Vielzahl von Abschnitten (111, 121, 131) unterteilt ist; die Größe des alphanumerischen Zeichens (14) und/oder des Symbols sowie die Größe des jeweiligen Radius der konzentrischen Kreise (11, 12, 13) werden anhand der Entfernung zwischen dem Patienten und der Optotype festgelegt.

8. Methode nach einem oder mehreren der vorstehenden Ansprüche 6 und 7,
bei der ein oder mehrere Schritte zum Austauschen von mindestens einer prismatischen Linse mit prismatischen Linsen mit abweichenden Dioptrien vorgesehen sind;
nach jedem Austauschen ist ein weiterer Prüfschritt mit der verwendeten Linse mit abweichender Dioptrie vorgesehen.

9. Set für die qualitative und quantitative Untersuchung des parazentralen Gesichtsfelds, für die Bestimmung aus einer Entfernung der PRLs und der Projektion der von Skotomen betroffenen Bereiche, das **dadurch gekennzeichnet ist, dass** es eine Linse nach Anspruch 1 umfasst und dass es ferner Prüfmittel beinhaltet, die zur Korrektur der Größe des Rotationswinkels des prismatischen Elements (21) dienen.

10. Ausbildungsmethode für die Suche und Fixation des exzentrischen bevorzugten Netzhautareals (PRL) innerhalb des parafovealen Bereichs, die **dadurch gekennzeichnet ist, dass** eine Linse, die nach einem oder mehreren der vorstehenden Ansprüche 1 oder 2 gefertigt wird, genutzt wird.

## Revendications

1. Verre destinée à une personne présentant une déficience visuelle au niveau d'un ou des deux yeux et comprenant au moins une partie de grossissement et au moins une partie prismatique ayant un élément prismatique prédéfini (21) et une orientation prédéfinie, ladite orientation correspondant à un angle de rotation ou de positionnement dudit élément prismatique autour d'un axe de rotation (A),
**caractérisé en ce que**
ledit élément prismatique (21) est et/ou peut être tourné selon un angle prédéfini autour d'un axe de rotation (A), ledit axe de rotation étant parallèle et/ou coïncidant avec l'axe perpendiculaire au plan anatomique frontal dudit verre (2),
ledit angle étant défini sur la base de la position du locus rétinien préféré (LRP) excentrique (31) dans la région parafovéale afin de faciliter la fixation de l'image sur ladite zone (31).

2. Verre selon la revendication 1, dans laquelle ladite au moins une partie prismatique comporte une graduation allant d'1 à 14 dioptries prismatiques, de préférence de 2 a 10 dioptries, notamment de 3 à 8 dioptries prismatiques.

3. Lunettes destinées à une personne présentant une déficience visuelle au niveau d'un ou des deux yeux, comprenant une paire de verres, **caractérisées en ce qu'**au moins un verre de ladite paire de verres est réalisé selon l'une ou plusieurs des revendications ou 2.

4. Lunettes selon la revendication 3, dans lesquelles ladite paire de verres est composée d'un premier verre et d'un second verre, ledit premier verre et ledit second verre étant composés d'une partie de grandissement (22) en association avec une partie prismatique (21), l'angle de rotation de 1"élément prismatique (21) dudit premier verre prismatique ayant une mesure différente par rapport à l' angle de rotation de l'élément prismatique (21) dudit second verre prismatique.

5. Lunettes selon la revendication 3 ou 4, dans lesquelles des moyens pour régler ledit élément prismatique (21) sont prévus.

6. Procédé d'évaluation qualitative et quantitative du champ visuel paracentral, pour l'identification des loci rétiniens préférés (LRPs) excentriques de loin et de la projection des zones scotomateuses au moyen d'au moins un verre selon la revendication 1, **caractérisé par** ailleurs en ce que l'évaluation qualitative et quantitative du champ visuel paracentral, l'identification des LRPs et la projection des zones scotomateuses sont effectuées en tournant le dit élément prismatique (21) autour d'un axe de rotation (A).

7. Procédé selon la vocation 6, dans lequel une étape de contrôle est prévue, pour vérifier que ledit élément prismatique (21) ait été tourné de manière à faciliter la fixation excentrique et donc à augmenter l'acuité visuelle du patient, ladite étape de contrôle étend effectuée par l'emploi d'un optotype composé d'un élément circulaire présentant une représentation graphique (1) composée d'une pluralité de cercles concentriques (11, 12, 13) et d'un caractère alphanumérique (14) et/ou un symbole situé au centre desdits cercles concentriques (11, 12, 13), une pluralité de lignes (15, 16, 17) étant agencées le long des rayons desdits cercles concentriques (11, 12, 13) à des intervalles réguliers, de façon que chaque cercle concentrique soit divisé en une pluralité de secteurs (111, 121, 131), la dimension dudit caractère alphanumérique (14) et/ou dudit symbole, aussi bien que la dimension de chaque rayon desdits cercles concentriques (11, 12, 13) étant définies sur la base de la distance entre le patient et ledit optotype.

8. Procédé selon l'une ou plusieurs des revendications précédentes 6 et 7, dans lequel une ou plusieurs étapes sont prévues pour remplacer ledit au moins un verre prismatique avec des verres prismatiques ayant un dioptre différent, une étape de contrôle additionnelle étant prévue après chaque étape de remplacement, en utilisant de verre avec le dioptre différent.

9. Kit pour l'évaluation qualitative et quantitative du champ visuel paracentral, pour l'identification des LRPs de loin et de la projection des zones scotomateuses, **caractérisé en ce qu'**il comporte un verre selon la revendication 1, et comprend par ailleurs des moyens de contrôle destinés à corriger la mesure de l'angle de rotation dudit élément prismatique (21).

10. Procédé d'entraînement pour la recherche et la fixation des loci rétiniens préférés (LRPs) excentriques dans la région parafovéale, **caractérisé en ce qu'**il comporte l'emploi d'un verre réalisé selon l'une ou plusieurs des revendications précédentes 1 et 2.
